# EUROPÄISCHE PATENTANMELDUNG

(11) **EP 4 349 287 A1**
(43) Veröffentlichungstag der Anmeldung: **10.04.2024**
(21) Anmeldenummer: 22200312.1
(22) Anmeldetag: 07.10.2022
(51) Int. Cl.: A61B 18/14

(54) **ABLATIONSINSTRUMENT**

(71) Anmelder: Erbe Elektromedizin GmbH, 72072 Tübingen (DE)
(72) Erfinder: ANDEL, Hanna, 72116 Moessingen (DE); ADLER, Marcus, 72393 Burladingen (DE); KARCHER, Felix, 72072 Tuebingen (DE)
(74) Vertreter: Rüger Abel Patentanwälte PartGmbB

(57) **Zusammenfassung**

Ein erfindungsgemäßes Ablationsinstrument (10) zeichnet sich durch mindestens eine, vorzugsweise mehrere Elektroden (21, 22) aus, die nach Art von Schraubenfedern ausgebildet sind und deren zum elektrischen Anschluss dienende Enden als axiale Fortsätze ausgebildet sind. Die Elektroden (21, 22) sitzen auf einem als Träger dienenden Innenschlauch (13), wobei die Fortsätze auf einem geringeren Abstand zur Längsachse liegen, als die Windungen der Elektrode. Zwischen dem jeweiligen Fortsatz (26) und der betreffenden Windung (25) ist ein Windungsabschnitt (27) ausgebildet, in dem sich der Abstand zur Längsmittelachse von dem Radius (R) auf einen geringeren Wert verringert, sodass der Fortsatz (26) bezüglich der Windungen (23, 24, 25) innen, d.h. näher an der Mittelachse (L) liegt. Zur Aufnahme dieses Fortsatzes und des zugehörigen elektrischen Anschlusses weist der Innenschlauch (13) eine Rinne (28) auf. Insgesamt ergibt sich ein zuverlässiger, flexibler und robuster Aufbau.

## Beschreibung

Die Erfindung betrifft ein Ablationsinstrument, insbesondere ein flexibles Ablationsinstrument, insbesondere zur Koagulation von Gewebe bei der sogenannten Radiofrequenzablation.

Ablationssonden dieser Art sind grundsätzlich bekannt, wie aus der EP 0 754 075 B1, der WO 94/11059, der EP 2 768 563 B1 oder der EP 1 181 896 A1 ersichtlich ist. Der Einsatz derartiger Instrumente bei der Koagulation von Lungentumoren ist aus der EP 3 763 314 A1 zu entnehmen.

Bei der Radiofrequenzablation wird die Ablationssonde mit ihrem aktiven distalen Ende direkt in der zu behandelnden Gewebestruktur platziert. Das Gewebe ist beispielsweise Lungen-, Leber- oder anderes Gewebe, in dem sich behandlungsbedürftiges Gewebe, z.B. Tumore, befinden. An dem distalen Ende der Sonde ist eine oder sind mehrere Elektroden vorhanden, die mit hochfrequentem Wechselstrom versorgt werden. Dieser erzeugt in dem Gewebe eine Thermonekrose. Die Elektroden können zur Vermeidung einer unerwünschten Erwärmung aktiv gekühlt sein. Die EP 2 309 941 B1 und die EP 3 769 706 A1 schlagen dazu vor, das distale Ende einer Ablationssonde mit einer internen Kühlung zu versehen.

Insbesondere bei der Behandlung von Lungengewebe ist es erforderlich, das Ablationsinstrument durch den Bronchialbaum mit immer kleiner werdenden Bronchien bis zu dem behandlungsbedürftigen Gewebe zu führen. Um dabei praktisch beliebige Partien des Lungenparenchyms erreichen zu können, ist zum einen ein sehr geringer Durchmesser des Ablationsinstruments und zum anderen eine möglichst hohe Flexibilität desselben zu wünschen, sodass möglichst enge Biegeradien, im Idealfall von weniger als 15 mm, und große Abwinklungen, im Idealfall von mehr als 145°, erreicht werden können. Andererseits soll die Sonde steif genug sein, um ein Einstechen in das betreffende zu behandelnde Gewebe zu ermöglichen. Weiter ist eine ausreichende mechanische Festigkeit erforderlich, um den Belastungen der Anwendung, insbesondere auftretenden Zugkräften und im Instrument durch die Kühlung hervorgerufenem Druck, widerstehen zu können. Ähnliche Probleme können sich auch bei anderen Geweben ergeben, die nur durch Endoskopie erreichbar sind.

Daraus leitet sich die der Erfindung zugrunde liegende Aufgabe ab, ein Konzept anzugeben, in dem sich eine zugleich hochflexible und mechanisch stabile Ablationssonde schaffen lässt.

Diese Aufgabe wird mit der Ablationssonde nach Anspruch 1 gelöst:
Das erfindungsgemäße Ablationsinstrument weist eine flexible Schlauchanordnung auf, auf der zumindest eine erste Elektrode angeordnet ist. Im bevorzugten Fall kann auf der Schlauchanordnung mindestens eine weitere Elektrode angeordnet sein. Die Elektrode ist nach Art einer Schraubenfeder ausgebildet und weist somit mehrere Windungen auf, die sich mit konstantem Radius um eine Mittelachse windend und einer Schraubenlinie folgend angeordnet sind. Die Mittelachse legt eine Längsrichtung fest, die mit der Längsrichtung der flexiblen Schlauchanordnung übereinstimmt. Zumindest eine endständige Windung der Elektrode ist mit einem Fortsatz verbunden, der zur elektrischen Kontaktierung dient und sich von der übrigen Elektrode weg erstreckt. Er ist dabei parallel oder in einem spitzen Winkel zu der Längsrichtung, d.h. zu der Mittelachse orientiert.

Zwischen dem sich im Wesentlichen axial erstreckenden Fortsatz und der mit diesem verbundenen Windung ist ein Windungsabschnitt angeordnet, der einerseits weiter in Umfangsrichtung orientiert ist, dabei aber einen zu dem Fortsatz hin abnehmenden Radius (Abstand zur Mittelachse) aufweist. Durch diese Formgebung kann der elektrische Anschluss der Elektrode unterhalb eines Isolatorkörpers vorgenommen werden, der sich nicht über den Radius der Elektrode hinaus erstreck. Ebenso kann der elektrische Anschluss unter einer benachbarten Elektrode oder auch proximal zu dieser liegen. Es wird dadurch erreicht, dass kein Teil des Instruments über die (in gestreckter Form zylindrische) Außenkontur des Ablationsinstruments hervorsteht und zwar auch dann nicht, wenn das Instrument mit kleinem Biegeradius stark abgewinkelt ist. Die Elektroden stellen keine steifen Stellen des Instruments dar, d.h. sie mindern dessen Flexibilität nicht wesentlich. Beim Abwinkeln des Instruments im Elektrodenbereich werden keine Teile der Elektroden oder sonstige Teile des Instruments nach außen gedrängt, insbesondere auch nicht die endständige Windung.

Die flexible Schlauchanordnung umfasst vorzugsweise einen Innenschlauch und einen darauf angeordneten Mantel. Der Innenschlauch kann z.B. aus dem Kunststoff PEEK bestehen. Er trägt in einem von dem Mantel befreiten Bereich eine oder mehrere Elektroden, wobei der Innenschlauch keine Unterbrechung für die Stromzufuhr zu den Elektroden aufweist. Vielmehr ist er unterbrechungsfrei (d.h. ohne irgendwelche die Wandung durchsetzenden Öffnungen) ausgebildet. Die Stromzuführungsleitung jeder Elektrode kann zwischen dem Mantel und dem Innenschlauch angeordnet sein. Zwischen dem Mantel und dem Innenschlauch kann dazu ein Spalt vorgesehen sein. Der Mantel kann z.B. aus dem Kunststoff PA ausgebildet sein. Außerdem kann der Kunststoff mit einem Gleitadditiv versehen sein. Dadurch kann eine relative Längsbewegung zwischen dem Innenschlauch und dem Mantel ermöglicht werden. Außerdem kann das Gleitadditiv das Einführen des Instruments in den Arbeitskanal eines Endoskops erleichtern.

Der Innenschlauch kann zur Aufnahme des Fortsatzes der Elektrode sowie von Verbindungsmitteln, die den Fortsatz mit einer elektrischen Leitung verbinden, einen Bereich aufweisen, in dem sein Querschnitt von dem ansonsten kreisförmigen Querschnitt abweicht. Der Innenschlauch kann dazu an wenigstens einer Stelle eine radial nach innen gerichtete Vertiefung aufweisen, die beispielsweise eine längsverlaufende Rinne bildet. Diese kann in dem entsprechenden Schlauchbereich durch eine Prägebearbeitung eingedrückt sein.

Sind im Längsabstand zueinander zwei Elektroden vorhanden, kann zwischen diesen ein Isolator angeordnet sein. Vorzugsweise weist dieser an mindestens einer Stirnseite, vorzugsweise an beiden Stirnseiten, eine einer Schraubenlinie folgende Stirnfläche auf. Dadurch ist es möglich, dass die jeweils letzte Windung der schraubenfederförmigen Elektrode an der Stirnseite des Isolators spaltfrei anliegt, sodass ein homogener und kantenfreier Übergang zwischen der Elektrode und dem Isolator gewährleistet ist. Dies führt zu einem optimierten Einführverhalten des Instruments in ein entsprechendes Zugangssystem, wie ein Endoskop oder dergleichen. Das Instrument kann leicht in den Arbeitskanal eingefädelt werden und hineingleiten, ohne irgendwo hängen zu bleiben.

Der Isolator kann außerdem eine Ausnehmung zur Aufnahme des Fortsatzes der Elektrode aufweisen, sodass der Fortsatz, ausgehend von dem Windungsabschnitt, ohne scharfen Knick in die in dem Innenschlauch ausgebildete Rinne eintauchen kann. Außerdem wird dadurch die Biegesteifigkeit des Instruments gemindert, die ansonsten durch den Fortsatz und die elektrische, z.B. durch eine Quetschhülse gebildete Verbindung zu einer Leitung erhöht sein könnte. Die Quetschhülse kann in Anschluss an die Elektrode oder auch in größerem axialen Abstand proximal uzu dieser angeordnet sein. Durch die in dem Isolator ausgebildete Ausnehmung kann eine geringe radiale Beweglichkeit des Fortsatzes und des Windungsabschnitts erreicht werden, was der Flexibilität des Instruments zugutekommt.

Zusätzlich kann der Isolatorkörper eine weitere Ausnehmung aufweisen, die als Klebstoffreservoir dient. Der Klebstoff kann zur Sicherung des Isolators und/oder der Elektrode auf dem Innenschlauch dienen.

Weiter Einzelheiten vorteilhafter Ausführungsformen der Erfindung sind Gegenstand von Unteransprüchen, der Zeichnung oder der Beschreibung.

### Es zeigen:

Figur 1 ein erfindungsgemäßes Ablationsinstrument, angeschlossen an ein schematisch dargestelltes versorgendes Gerät, in perspektivischer Übersichtsdarstellung,
Figur 2 das distale Ende des Ablationsinstruments, in längsgeschnittener und schematisierter nicht maßstäblicher Darstellung,
Figur 3 einen Querschnitt des Instruments nach Figur 2 geschnitten entlang der Linie III-III,
Figur 4 einen Querschnitt des Instruments nach Figur 2 geschnitten entlang der Line IV-IV,
Figur 5 eine Stirnansicht der Elektrode des Instruments nach Figur 1 und 2, in nicht maßstäblicher Darstellung,
Figur 6 eine Ausschnittsansicht des Instruments nach Figur 1 mit zwei Elektroden und einem dazwischen angeordneten Isolatorkörper,
Figur 7 eine durch eine Quetschhülse gebildete elektrische Verbindung zwischen dem Fortsatz der Elektrode und einer elektrischen Leitung.

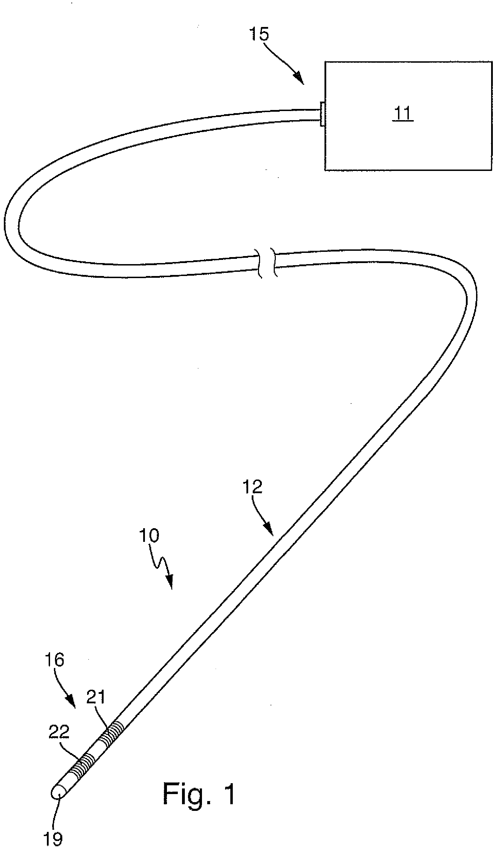

In Figur 1 ist ein Instrument 10 in Gestalt eines Radioablationsinstruments veranschaulicht, das insbesondere zur Behandlung von Gewebe in der Lunge, prinzipiell aber auch zur Behandlung anderer Gewebearten vorgesehen und geeignet ist. Das Instrument 10 wird typischerweise durch ein Endoskop in den Bronchialbaum der Lunge eingeführt, um dann dort gezielt in behandlungswürdiges Gewebe eingestochen zu werden. Ein entsprechendes Endoskop sowie der zu behandelnde Patient sind in Figur 1 nicht dargestellt. Das Instrument 10 ist an ein speisendes Gerät 11 oder Gerätesystem angeschlossen, das in Figur 1 lediglich schematisch angedeutet ist. Es dient zur Versorgung des Instruments 10 mit Kühlfluid und mit elektrischem Strom zur Behandlung des Gewebes.

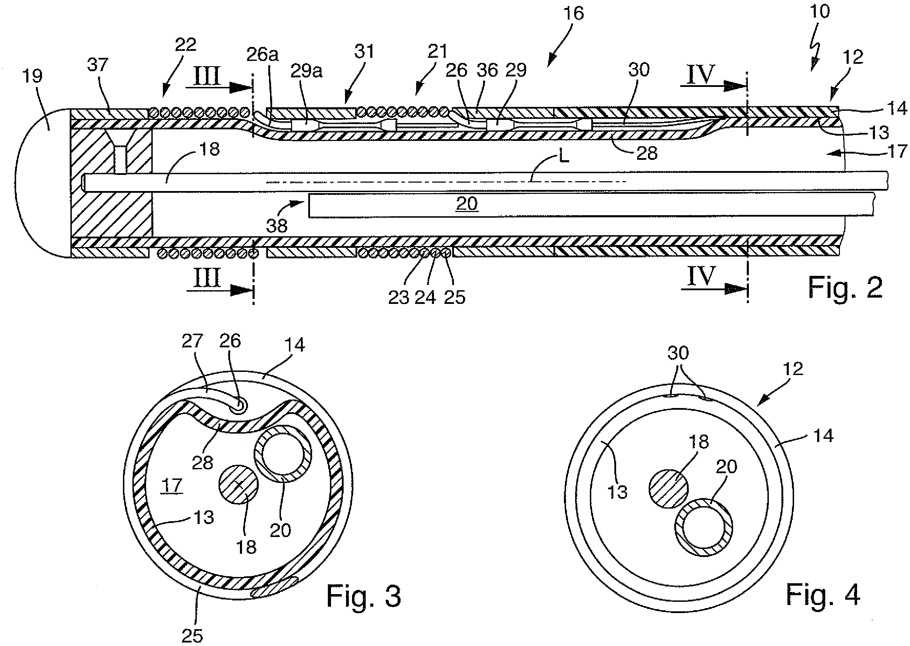

Das Instrument 10 weist eine Schlauchanordnung 12 auf, deren Aufbau insbesondere aus den Figuren 2 bis 4 ersichtlich ist. Zu der Schlauchanordnung 12 gehören ein Innenschlauch 13 und ein darauf angeordneter Mantel 14. Der Innenschlauch 13 und der Mantel 14 sind jeweils aus Kunststoff, vorzugsweise unterschiedlichen Kunststoffen ausgebildet. Der Innenschlauch ist vorzugsweise aus einem plastisch verformbaren Kunststoff, z.B. PEEK ausgebildet, in den durch Prägen Strukturen, wie Rillen, Vertiefungen oder dergleichen ausbildbar sind. Der Mantel ist vorzugsweise aus einem gleitfähigen Kunststoff, z.B. PA ausgebildet. Er kann dazu mit einem Gleitmittelzusatz versehen sein.

Von seinem proximalen Ende 15 (Figur 1) bis zu seinem distalen Ende 16 erstreckt sich durch den Innenschlauch ein Lumen 17. Durch das Lumen 17 erstreckt sich ein Zugdraht 18, dessen distales Ende mit einem Verschlussstück 19 verbunden ist, das das Lumen 17 endseitig abschließt. Der Zugdraht 18 kann mit dem Verschlussstück 19 stoffschlüssig verbunden, insbesondere verklebt oder verschweißt sein.

Das Verschlussstück 19 kann einen distal gerundeten Kopf und einen Schaft aufweisen, der sich in den Innenschlauch 13 hinein erstreckt. Das Verschlussstück 19 kann aus Kunststoff, Keramik oder Metall ausgebildet sein und bildet das distale Ende des Instruments 10. Das Verschlussstück 19 kann auch aus mehreren Bauteilen bestehen, beispielsweise einem Isolatorkörper mit einem oder mehreren darin angeordneten Metallteilen, die als Elektrode dienen können. Mit dieser gegebenenfalls über den Zugdraht 18 bestromten Elektrode wird das Einstechen des Instruments in das Gewebe erleichtert. Alternativ kann das Verschlussstück als massives Metallteil ausgebildet sein, das außen mit einer isolierenden Beschichtung versehen ist. Am distalen Ende frei liegende Stellen können als Elektrode dienen und, wenn sie z.B. über den Zugdraht 18 bestromt werden, das Einstechen des Ablationsinstruments in das Gewebe erleichtern.

Der Zugdraht 18 kann aus einem zugfesten Metall, z.B. Nitinol ausgebildet sein. Er dient zur Übertragung von Zugkräften in dem Instrument 10, um dieses sicher aus dem Gewebe herausziehen zu können. Der Zugdraht 18 kann zugleich als elektrische Leitung dienen, wenn das Verschlussstück 19 zusätzlich eine elektrische Funktion als Elektrode übernehmen soll. Außerdem unterstütz der vorzugsweise federsteife Zugdraht 18, bspw. aus Nitinol, die Formstabilität des Ablationsinstruments 10. Gerade nach Abwinkelung des Ablationsinstruments mit engen Biegeradien ist das Ablationsinstrument nicht bleibend verformt, sondern findet selbständig in seine gestreckte Form zurück.

In dem Lumen 17 ist außerdem ein Zuführungsschlauch 20 angeordnet, der sich von dem proximalen Ende 15 bis in das distale Ende 16 hinein erstrecken kann und dazu dient, das distale Ende 16 des Instruments 10 mit kühlendem Fluid zu versorgen. Der Zuführungsschlauch kann eine oder mehrere Öffnungen aufweisen, über die das Kühlfluid gezielt in dem distalen Ende 16 des Instruments 10 freigesetzt wird. Das Kühlfluid kann dann über das Lumen zu dem Gerät 11 zurückgeführt werden.

Auf dem Innenschlauch 13 ist in einem vorzugsweise von dem Mantel 14 befreiten Abschnitt des distalen Endes 16 zumindest eine erste Elektrode 21 angeordnet, die der Bestromung des zu behandelnden Gewebes dient. Optional kann auf dem Innenschlauch 13 in distalem Abstand zu der ersten Elektrode eine weiteren Elektrode 22 vorgesehen sein, die vorzugsweise mit der ersten Elektrode 21 identisch aufgebaut ist. Die nachfolgende Beschreibung der ersten Elektrode 21 gilt somit entsprechend ohne gesonderte Erwähnung auch für die Elektrode 22. Weitere solche Elektroden können bedarfsweise vorgehsehen sein.

Die Elektrode 21 wird durch einen elektrischen Leiter gebildet, der einer Schraubenlinie folgend geformt ist. Die Elektrode 21 kann durch einen Federdraht, eine mit einem schraubenförmigen Schlitz versehene Hülse, z.B. aus Edelstahl, oder dergleichen gebildet und bedarfsweise mit einer Beschichtung, z. B. einer Silber- oder Goldbeschichtung versehen sein. Beispielsweise kann die Elektrode 21 die Form einer Schraubenfeder mit mehreren Windungen 23, 24, 25 aufweisen. Zur elektrischen Kontaktierung der Elektrode 21 geht deren letzte Windung 25, die vorzugsweise die proximale Windung ist, in einen Fortsatz 26 über, der sich im Wesentlichen in Längsrichtung des Instruments 10 erstreckt. Die Längsrichtung des Instruments 10 ist in Figur 2 durch eine strichpunktierte Linie L angedeutet. Diese strichpunktierte Linie L ist zugleich die Mittelachse einer Schraubenlinie, der die Windungen 23, 24, 25 folgen. Zugleich markiert die Längsachse L die Längsrichtung des Instruments 10 (von distal nach proximal) und somit auch die Längsrichtung der Schlauchanordnung 12 und somit des Innenschlauchs 13 und des Mantels 14.

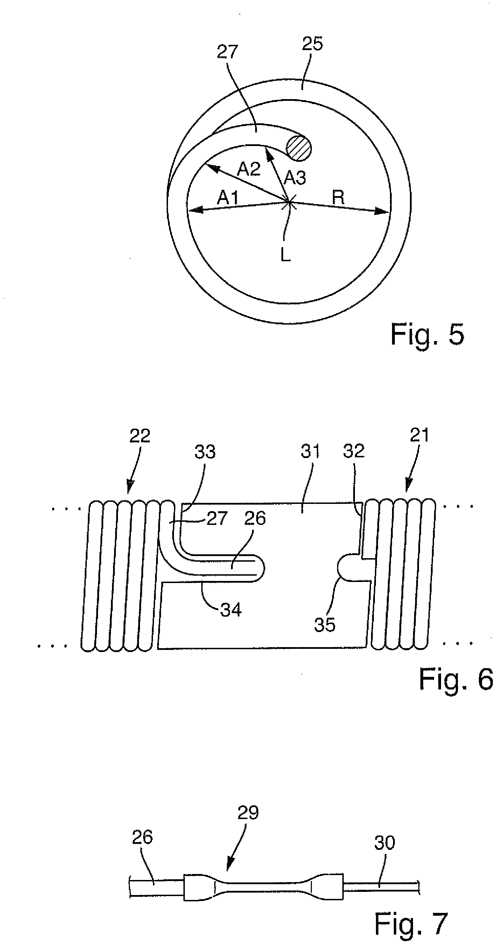

Zwischen dem vorzugsweise parallel zur Längsrichtung L (oder in einem spitzen Winkel zu dieser) angeordneten Fortsatz 26 und der der Schraubenlinie folgenden Windung 25 liegt ein Windungsabschnitt 27, der sich in Verlängerung der Windung 25 in Umfangsrichtung erstreckend radial nach innen abgebogen ist. In den Figuren 3 und 5 ist dies zur Verdeutlichung etwas überhöht veranschaulicht. Während die Windungen 23, 24, 25 einen konstanten Radius R aufweisen, verringert sich der Abstand von einem großen nahezu mit dem Radius R übereinstimmenden Abstand A1 zu einem kleinerem Abstand A2 und zu einem noch kleineren Abstand A3 zwischen dem jeweiligen Bereich des Windungsabschnitts 27 und der Mittellinie L. Der Windungsabschnitt 27 erstreckt sich über einen Teil des Umfangs der Elektrode, vorzugsweise wenigstens über zehn Grad oder mehr.

Zumindest im Bereich des Fortsatzes 26 weist der Innenschlauch 13 einen nach innen deformierten Rinnenabschnitt 28 auf, in dem der elektrische Anschluss der Elektrode 21 angeordnet ist. Dieser Anschluss wird durch ein geeignetes Verbindungsmittel, beispielsweise eine Crimphülse 29, gebildet. Die Crimphülse 29 ist ein elektrisch leitendes Röhrchen, in dessen einen Ende der Fortsatz 26 und in dessen anderen Ende eine elektrische Leitung 30 steckt, wie es Figur 7 zeigt. Die Crimphülse erstreckt sich im Wesentlichen in Längsrichtung L. Sie kann eine Steifigkeit aufweisen, die größer ist als die Steifigkeit der Leitung 30 oder des Fortsatzes 26.

Die elektrische Leitung 30 erstreckt sich von der Crimphülse 29 bis zu dem proximalen Ende 15 des Instruments 10 und ist dort elektrisch mit einem nicht weiter veranschaulichten Generator verbunden. Die Leitung 30 hat dabei, wie Figur 4 zeigt, ihren Platz in einem Spalt zwischen dem Mantel 14 und dem Innenschlauch 13. Sind mehrere Elektroden 21, 22 vorhanden, sind auch entsprechend mehrere solcher Leitungen 30 vorgesehen. Die Leitungen 30 können beispielsweise als Lackdrähte, zum Beispiel Kupferlackdrähte, Silberlackdrähte oder andere geeignete Leitungen gebildet sein. Die Crimphülse 29 kann zum Beispiel mittels eines Schrumpfschlauchs an ihrer Außenseite elektrisch isoliert sein.

Wie Figur 2 zeigt, kann sich die Crimphülse 29a der zweiten Elektrode 22 in der Rinne 28 liegend unter die Elektrode 21 erstrecken. Ein elektrischer Kurzschluss wird durch entsprechende Isolierung der Crimphülse 29a wirksam verhindert. Die Rinne 28 kann in Radialrichtung eine Tiefe aufweisen, die größer ist als der Außendurchmesser der Crimphülse 29a, so dass die Crimphülse 29a in der Rinne eine gewisse Bewegungsfreiheit hat. Alternativ kann die Crimphülse 29a bei entsprechend langer Ausführung des Fortsatzes 26a auch proximal zu der ersten Elektrode 21 in der Rinne 28 positioniert sein. Sowohl die Crimphülsen 29, 29a als auch die Fortsätze 26, 26a sind mit geeignetem Isolationsmaterial, z. B. Schrumpfschlauch versehen.

Zwischen den beiden Elektroden 21, 22 kann ein Isolator 31 vorgesehen sein, der beispielsweise aus einem steifen oder auch einem flexiblen Kunststoff ausgebildet sein kann. Der Isolator 31 kann, wie Figur 6 zeigt, Stirnflächen 32, 33 aufweisen, die eine an die Kontur der jeweiligen Elektrode 21, 22 angepasste Form haben. Die Stirnflächen 32, 33 folgen somit der Steigung der Windungen der beiden Elektroden 21, 22. Dadurch wird die Ausbildung von offenstehenden Spalten der Stufen zwischen der Elektrode 21 und dem Isolator 31 ebenso verhindert, wie zwischen dem Isolator 31 und der Elektrode 22. Außerdem kann der Isolator 31 eine sich von der Stirnfläche 33 weg erstreckende Ausnehmung 34 zur Aufnahme des Fortsatzes 26 aufweisen. Damit wird verhindert, dass der Isolator den Fortsatz 26 radial nach innen und damit den gegenüber liegenden Teil der anschließenden Windung radial nach außen drückt. Außerdem wird die Flexibilität des Instruments 10 in diesem Bereich verbessert. Die Ausnehmung 34 kann dem mit der Crimphülse 29a verbundenen Fortsatz 26 eine radiale Bewegungsfreiheit gewähren, so dass sich deren Steifigkeit nicht auf das Instrument überträgt.

Eine weitere Ausnehmung 35, die von der Stirnfläche 32 ausgeht, kann als Klebstoffreservoir genutzt werden, um den Isolator 31 und/oder die Elektrode 21 auf dem Innenschlauch 13 zu fixieren.

Das Instrument kann einen Zentrierkörper 36 zwischen der ersten Elektrode 21 und dem Mantel 14 aufweisen. Dieser Zentrierkörper 36 kann an seiner der Elektrode 21 zugewandten Stirnseite entsprechend der Stirnseite 33 des Isolators 31 nach Figur 6 ausgebildet sein. An seiner von der Elektrode 21 abliegenden Seite kann er eine ringförmige, ebene Stirnfläche aufweisen, mit der er an den Mantel angrenzt. Der Zentrierkörper 36 kann als geschlitzter Metallring ausgebildet sein, der einen definierten Ringspalt zwischen dem Innenschlauch14 und dem Mantel 14 sicherstellt.

Ebenso kann zwischen dem Verschlussstück 19 und der zweiten Elektrode 22 ein Isolator 37 angeordnet sein, der mit mindestens einer ringförmig angeordneten Aussparung, ebenen Stirnfläche an das Verschlussstück 19 anstößt. An seiner der Elektrode 22 zugewandten Stirnseite kann er entsprechend der Stirnseite 32 des Isolators 31 nach Figur 6 ausgebildet sein.

Das insoweit beschriebene Instrument 10 arbeitet wie folgt:
Im medizinischen Einsatz wird das Instrument 10 endoskopisch in den Patienten eingeführt und das distale Ende 16 in das zu behandelnde Gewebe so eingestochen, dass die Elektrode 21 Gewebekontakt hat. Hat das Instrument 10 zwei Elektroden 21, 22, wird es so eingestochen, dass beide Elektroden 21, 22 Gewebekontakt haben. Das Gerät 11 gibt dann Strom an die eine oder die beiden Elektroden 21, 22 ab, der das zu abladierende Gewebe durchströmt. Gleichzeitig wird über den Versorgungsschlauch 20 Kühlfluid in das distale Ende 16 des Instruments 10 geliefert, um die Elektroden 21, 22 zu kühlen. Verbrauchtes Kältefluid strömt durch das Lumen 17 zu dem Gerät 11 zurück.

Das Instrument 10 ist hochflexibel ausgebildet. Beim endoskopischen Einführen in den Patienten kann es engen Biegeradien folgen. Es kann dabei insbesondere im Bereich seines distalen Endes 16 auch unter Verformung der Elektroden 21, 22 abgewinkelt werden, ohne dass dabei einzelne Windungen über die kreisförmige Außenkontur des Instruments 10 hinausgedrängt werden. Die Steifigkeit der Crimphülse 29a ist von dem Instrument 10, insbesondere von der Schlauchanordnung 12, weitgehend, d.h. im Rahmen der gewünschten Biegeradien, entkoppelt. Damit sind auch ungünstig gelegene Tumore oder sonstiges behandlungsbedürftiges Gewebe gut erreichbar. Außerdem lassen sich mit dem vorgestellten Konzept Instrumente mit einem Außendurchmesser von weniger als 2 mm bereitstellen, wodurch das Instrument 10 sehr tief auch in enge Strukturen eingeführt werden kann. Zudem ist der Innenschlauch 13 unterbrechungsfrei. Die elektrischen Leitungen 30 durchdringen ihn an keiner Stelle. Sie werden vielmehr in dem Spalt zwischen dem Innenschlauch 13 und dem Mantel 12 geführt. Dies erhöht die Leckagesicherheit, da keine Dichtstellen im biegebelasteten Bereich vorliegen. Auch dadurch ist das Instrument 10 sicher gegen Fehlfunktion. Das erfindungsgemäße Instrument 10 weist einen hohen Grad an Patientensicherheit auf.

Ein erfindungsgemäßes Ablationsinstrument 10 zeichnet sich durch mindestens eine, vorzugsweise mehrere Elektroden 21, 22 aus, die nach Art von Schraubenfedern ausgebildet sind und deren jeweilige zum elektrischen Anschluss dienenden Enden als axiale Fortsätze ausgebildetsind. Die Elektroden 21, 22 sitzen auf einem als Träger dienenden Innenschlauch 13, wobei die Fortsätze auf einem geringeren Abstand zur Längsachse liegen, als die Windungen der Elektrode. Zwischen dem jeweiligen Fortsatz 26 und der betreffenden Windung 25 ist ein Windungsabschnitt 27 ausgebildet, in dem sich der Abstand zur Längsmittelachse von dem Radius R auf einen geringeren Wert verringert, sodass der Fortsatz 26 bezüglich der Windungen 23, 24, 25 innen, d.h. näher an der Mittelachse L liegt. Zur Aufnahme dieses Fortsatzes und des zugehörigen elektrischen Anschlusses weist der Innenschlauch 13 eine Rinne 28 auf. Insgesamt ergibt sich ein zuverlässiger, flexibler und zugleich robuster Aufbau.

### Bezugszeichen:

- 10: Instrument
- 11: Gerät
- 12: Schlauchanordnung
- 13: Innenschlauch
- 14: Mantel
- 15: proximales Ende
- 16: distales Ende
- 17: Lumen
- 18: Zugdraht
- 19: Verschlussstück
- 20: Zuführschlauch
- 21: erste Elektrode
- 22: weitere Elektrode
- 23 - 25: Windungen
- L: Längsrichtung
- M: Mittellinie / Mittelachse
- 26: Fortsatz
- 27: Windungsabschnitt
- 28: Rinnenabschnitt
- 29: Crimphülse
- 30: Leitung
- 31: Isolator
- 32, 33: Stirnflächen
- 34, 35: Ausnehmungen
- 36: Zentrierkörper
- 37: Isolator
- 38: Auslassöffnung

## Patentansprüche

1. Ablationsinstrument (10), insbesondere zur HF-Ablation,
mit einer flexiblen Schlauchanordnung (12), auf der eine erste Elektrode (21) angeordnet ist,
wobei die Elektrode (21) mehrere Windungen (23, 24, 25) aufweist, die sich mit konstantem Radius (R) um eine Mittelachse (M) windend, einer Schraubenlinie folgend angeordnet sind, wobei die Mittelachse (M) eine Längsrichtung (L) festlegt,
wobei eine endständige Windung (25) mit einem Fortsatz (26) verbunden ist, der sich von der Elektrode (21) weg erstreckend zu der Längsrichtung (L) parallel oder in einem spitzen Winkel zu dieser angeordnet ist,
wobei zwischen der Windung (25) und dem Fortsatz (26) ein Windungsabschnitt (27) angeordnet ist,
**dadurch gekennzeichnet,**
**dass** zwischen der Mittelachse (M) und der dem Windungsabschnitt (27) ein Abstand (A) festgelegt ist, der von der Windung (25) zu dem Fortsatz (26) hin abnimmt.

2. Ablationsinstrument nach Anspruch 1, **dadurch gekennzeichnet, dass** die Schlauchanordnung (12) mindestens einen Innenschlauch (13) und mindestens einen darauf angeordneten Mantel (14) aufweist.

3. Ablationsinstrument nach Anspruch 2, **dadurch gekennzeichnet, dass** der Innenschlauch (13) einen kreisförmigen Querschnitt aufweist.

4. Ablationsinstrument nach Anspruch 2 oder 3, **dadurch gekennzeichnet, dass** der Innenschlauch (13) an wenigstens einer Stelle eine radial nach innen gerichtete Vertiefung (28) aufweist.

5. Ablationsinstrument nach Anspruch 4, **dadurch gekennzeichnet, dass** die Vertiefung (28) mindestens teilweise unterhalb der ersten Elektrode (21) angeordnet ist.

6. Ablationsinstrument nach einem der vorstehenden Ansprüche, **dadurch gekennzeichnet, dass** in Längsrichtung (L) von der ersten Elektrode (21) beabstandet mindestens eine weitere Elektrode (22) vorgesehen ist.

7. Ablationsinstrument nach Anspruch 6, **dadurch gekennzeichnet, dass** die weitere Elektrode (22) in Übereinstimmung mit der ersten Elektrode (21) ausgebildet ist.

8. Ablationsinstrument nach Anspruch 6 oder 7, **dadurch gekennzeichnet, dass** zwischen den beiden Elektroden (21, 22) ein Isolator (31) angeordnet ist.

9. Ablationsinstrument nach Anspruch 8, **dadurch gekennzeichnet, dass** der Isolator (31) mindestens eine einer Schraubenlinie folgende Stirnfläche (32, 33) aufweist.

10. Ablationsinstrument nach Anspruch 8 oder 9, **dadurch gekennzeichnet, dass** der Isolator (31) hohlzylindrisch ausgebildet ist und eine Ausnehmung (34) zur Aufnahme des Fortsatzes (26) aufweist.

11. Ablationsinstrument nach einem der vorstehenden Ansprüche, **dadurch gekennzeichnet, dass** der Isolator (31) mindestens ein Klebstoffreservoir aufweist.

12. Ablationsinstrument nach Anspruch 11, **dadurch gekennzeichnet, dass** das Klebstoffreservoir durch eine stirnseitige Ausnehmung (34, 35) gebildet ist.

13. Ablationsinstrument nach einem der vorstehenden Ansprüche, **dadurch gekennzeichnet, dass** der Fortsatz (26) mit einer elektrischen Leitung (30) verbunden ist.

14. Ablationsinstrument nach Anspruch 13, **dadurch gekennzeichnet, dass** der Fortsatz (26) und die Leitung (30) mittels einer Quetschhülse (29) miteinander verbunden sind.

15. Ablationsinstrument nach einem der vorstehenden Ansprüche, **dadurch gekennzeichnet, dass** der Innenschlauch (13) ein Lumen (17) umgrenzt, in dem ein Versorgungsschlauch (20) mit wenigstens einer Auslassöffnung (38) angeordnet ist.
